# EUROPÄISCHE PATENTSCHRIFT

(11) **EP 1 411 051 B1**
(45) Veröffentlichungstag und Bekanntmachung des Hinweises auf die Patenterteilung: **14.06.2006**
(21) Anmeldenummer: 03018973.2
(22) Anmeldetag: 21.08.2003
(51) Int. Cl.: C07D 303/04, C07D 301/12

(54) **Verwendung von Verbindungen zur beschleunigten Phasentrennung bei heterogenen Epoxidierungsreaktionen cyclischer Alkene**
Use of compounds for accelerated phase separation in heterogenic epoxydation reactions of cycloalkenes
Usage de composés pour la séparation accelerée des phases dans des réactions hétérogènes d'époxydation de cycloalcenes

(30) Priorität: 11.10.2002 DE 10247496
(43) Veröffentlichungstag der Anmeldung: 21.04.2004
(73) Patentinhaber: Degussa AG, 40474 Düsseldorf (DE)
(72) Erfinder: Herwig, Jürgen, Dr., 46565 Hünxe (DE); Roos, Martin, Dr., 45721 Haltern am See (DE); Oenbrink, Georg, Dr., 48249 Dülmen (DE); Günzel, Bernd, Dr., 45721 Haltern am See (DE); Lohmar, Jörg, Dr., 44141 Dortmund (DE)

(56) Entgegenhaltungen:
- DE-A- 3 027 349

## Beschreibung

Die vorliegende Erfindung betrifft einen neuen Absetzbeschleuniger bei der Epoxidierung eines cyclischen, mindestens einfach ungesättigten Alkens.

Es sind zahlreiche Verfahren zur Epoxidierung von Alkenen bekannt und es kann eine große Bandbreite verschiedener Reaktions- beziehungsweise Katalysatorsysteme eingesetzt werden. Die Epoxidierung von Alkenen in homogener, flüssiger Phase mit organischen Hydroperoxiden in Anwesenheit von Katalysatoren auf der Basis Molybdän, Wolfram beziehungsweise Vanadium findet industrielle Anwendung.
Die Epoxidherstellung wird jedoch durch äquivalente oder sogar größere Mengen des dem Hydroperoxid entsprechenden Alkohols begleitet, dessen Verwertung oder Rückführung eine großtechnische Anwendung dieses Verfahrens stark einschränkt.

Aus diesem Grund wurden verstärkt direktere Oxidationsverfahren (Epoxidierung) von Alkenen entwickelt.

Ein entsprechendes Verfahren ist die Epoxidierung mittels molekularem Sauerstoff unter Verwendung von Silberkatalysatoren. Dieses Verfahren konnte aber nur bei Ethen erfolgreich angewendet werden; dieses Verfahren ließ sich nicht auf andere, interessante Alkene (wie zum Beispiel Propen) analog übertragen.

Ein anderes Verfahren für eine direkte Oxidation von Alkenen zu Epoxiden ist die Umsetzung mit Wasserstoffperoxid.
Dieses Verfahren ist vor allem aufgrund der positiven Eigenschaften des Oxidationsmittels in Bezug auf eine wesentlich geringere Umweltbelastung für verschiedene Epoxidationsverfahren vorgeschlagen worden. Da die Aktivität von Wasserstoffperoxid gegenüber den Alkenen nur gering ist, teilweise sogar völlig fehlt, müssen Aktivierungsmittel, gewöhnlich organische Säuren, wie Ameisensäure, Essigsäure usw., in organischen Lösemitteln verwendet werden, wobei die Säuren in Form von Persäuren das reaktionsfähige Epoxidationsmittel in situ bilden.

Auch diese Verfahren scheinen nicht besonders erfolgreich, und zwar weil es schwierig ist, die Persäuren zu erhalten und aufgrund der Instabilität der Epoxide in saurem Medium, wodurch recht mühsame Verfahrensbedingungen notwendig werden.
Ein weiteres Verfahren ist die Oxidation von Alkenen durch Reaktion mit hoch konzentriertem Wasserstoffperoxid in homogener, d. h. ausschließlich organischer, flüssiger Phase in Anwesenheit löslicher Katalysatorsysteme auf der Basis von Elementen der Gruppen IVb, Vb und VIb des Periodensystems (Ti, V, Mo, W) in Verbindung mit Elementen aus der Gruppe von Pb, Sn, As, Sb, Bi, Hg usw.
Auch hier lassen die Ergebnisse keine Übertragung auf ein industrielles Verfahren zu. Denn einerseits läuft die Reaktion langsam ab, andererseits ist die Herstellung der Katalysatorsysteme, die im Allgemeinen aus sehr komplizierten organischen Metallverbindungen bestehen und darüber hinaus in dem organischen Reaktionsmedium löslich sein müssen, aufwändig und teuer. Des weiteren bringt die Anwendung von hoch konzentriertem Wasserstoffperoxid (> 70 %) erhebliche Sicherheitsgefahren mit sich, die in wirtschaftlicher Weise nicht leicht überwunden werden können.

Diese Verfahren des Standes der Technik verdeutlichen, dass die Oxidation von Alkenen mit Wasserstoffperoxid in sich selbst widersprüchlich ist, weil die Arbeitsbedingungen bezüglich Katalysatorsystem und Wasserstoffperoxid bestenfalls ein wässriges, möglichst saures Medium erfordern, während die Oxidationsreaktion und die Stabilität des Epoxids vorzugsweise ein neutrales organisches Medium benötigen.

Daher wurden weitere Verfahren zur Epoxidierung von Alkenen mit Wasserstoffperoxid entwickelt, bei denen entweder ein verbessertes Katalysatorsystem auf Basis von TiO₂/SiO₂ in wässriger Phase unter Zusatz von primären oder sekundären Alkoholen (vgl. EP 0 987 259 Al) oder ein Zweiphasensystem mit einem Katalysator aus Wolframsäure, einem quartären Ammoniumsalz und einer Phosphorverbindung (zum Beispiel DE 30 27 349) zum Einsatz kommt.

Für Alkene, deren Epoxide nicht hydrolyselabil sind und bei denen die olefinische Doppelbindung nicht sterisch gehindert ist (wie z. B. cyclische mindestens einfach ungesättigte Alkene) ist die bekannte Epoxidierung mit Wasserstoffperoxid und einem Wolframkatalysator die wirtschaftlichste Alternative.

Damit die Epoxidierung mit Wasserstoffperoxid schnell genug verläuft, wird bei sehr lipophilen Alkenen (wie zum Beispiel Cyclododecen) meist ein Phasentransferkatalysator (zum Beispiel Aliquat® 336) verwendet (Angew. Chem. (1991), 103(12), 1706 - 9). Die gewünschte starke Beschleunigung der Epoxidierung durch den Phasentransferkatalysator führt jedoch dazu, dass die Phasen nach der Reaktion aufgrund von Emulsionsbildung deutlich schwieriger zu trennen sind; die entsprechenden Absetzzeiten erhöhen sich stark. Auch verbleibt die organische Phase nach der Trennung meistens stark getrübt. Um eine weitgehend vollständige Phasentrennung zu erreichen, müssen entweder Phasentrenner mit sehr großen Volumina oder geeignete Zentrifugen eingesetzt werden.
Durch die erhöhten Absetzzeiten dieses Verfahrens wird die Attraktivität für eine großtechnische, kontinuierliche Anwendung stark vermindert. Insbesondere eine Übertragung des Verfahrens auf bestehende Anlagen ist aufgrund der Notwendigkeit vergrößerter Phasentrenner meistens aufgrund von Platzproblemen gar nicht realisierbar. Die Verwendung von Zentrifugen ist angesichts der Stromkosten und des Wartungsaufwandes aufgrund bewegter Teile uninteressant.

Ganz allgemein kann gesagt werden, dass Absetzzeiten von weniger als 2 Minuten als technisch wünschenswert zu bezeichnen sind. Betragen die Absetzzeiten dagegen mehr als 4 Minuten, so kann ein großtechnischer kontinuierlicher Prozess nur schwer wirtschaftlich betrieben werden.

In DE 30 27 349 wird ein Verfahren zur Epoxidierung von Alkenen mit Wasserstoffperoxid, einer Wolframverbindung, einer Phosphorverbindung und einem Phasentransferkatalysator beschrieben. Bei diesem Verfahren werden zwingend Lösemittel verwendet, beispielsweise Alkane oder Cycloalkane. Diese Lösemittel werden immer in größeren Mengen der Reaktionsmischung zugesetzt und dienen im Allgemeinen dazu, entweder einen festen Stoff aufzulösen und so zur Reaktion zu bringen, oder die Reaktionsführung zu verbessern, beispielsweise um eine bessere Wärmeabfuhr zu gewährleisten.

Allerdings ist die Verdünnung von Edukten mit nicht reaktionsfähigen Stoffen, wie zum Beispiel Lösemitteln, nicht erwünscht, da einerseits die Verdünnung zu einer Erniedrigung der Raum-Zeit-Ausbeute führt und anderseits eine weitere Trennoperation nach der Reaktion erforderlich ist.

Die Aufgabe der vorliegenden Erfindung besteht in der Bereitstellung eines Absetzbeschleunigers, das heißt einer Verbindung, die bei der Epoxidierung von Alkenen zu technisch akzeptablen Absetzzeiten der heterogenen Katalysator-/Reaktionsmischung führt, wobei eine ausreichend hohe Raum-Zeit-Ausbeute an Epoxid gewährleistet ist, so dass dieses Verfahren unter Verwendung des Absetzbeschleunigers eine großtechnische Herstellung entsprechender Epoxide ermöglicht.

Die Aufgabe wird erfindungsgemäß gelöst durch die Verwendung eines cyclischen Alkans mit 8 bis 20 Kohlenstoffatomen im Cyclus als Absetzbeschleuniger bei der Epoxidierung eines cyclischen, mindestens einfach ungesättigten Alkens mit 8 bis 20 Kohlenstoffatomen im Cyclus, wobei als cyclisches Alkan die zu dem eingesetzten Alken korrespondierende, gesättigte Verbindung verwendet wird, dadurch gekennzeichnet, dass die Epoxidierung in Gegenwart von Wasserstoffperoxid als Oxidationsmittel und eines Katalysatorsystems durchgeführt wird, und wobei das Katalysatorsystem mindestens ein Metall der Gruppen IVb, Vb oder VIb des Periodensystems der Elemente, Phosphorsäure sowie einen Phasentransferkatalysator enthält, dass dieser Absetzbeschleuniger in einer Menge von mindestens 1 Gew.-%, besonders bevorzugt von mindestens 2,5 Gew.-%, in der Reaktionsmischung enthalten ist und dass das Reaktionsgemisch mindestens zwei flüssige Phasen enthält.

Die Angabe "Gew.-%" bedeutet im gesamten Anmeldetext der in Prozenten ausgedrückte, gewichtsmäßige Anteil der entsprechenden Komponente, bezogen auf das Epoxid.

Das Ergebnis des erfindungsgemäßen Gegenstands ist insofern überraschend, als dass die Anwesenheit des zum eingesetzten Olefins korrespondierenden Alkans bereits in geringen Mengen zu einer erheblichen Beschleunigung der Absetzzeiten und damit insgesamt zu einem wirtschaftlicheren Prozess führt.
Im Gegensatz zu den Epoxidierungsverfahren des Standes der Technik, die einen Phasentransferkatalysator verwenden, führt die erfindungsgemäße Verwendung des Absatzbeschleunigers zu Absetzzeiten der am Ende der Oxidationsreaktion resultierenden heterogenen Mischung aus Katalysator und Produkt von weniger als zwei Minuten.
Unter dem Begriff Absetzzeit wird die Zeit verstanden, nach der sich die Phasen vollständig getrennt haben. Die Phasentrennung wird definitionsgemäß als beendet angesehen, wenn die letzte Tropfenschicht nur noch die Hälfte der Phasengrenze bedeckt, die andere Hälfte als klare Grenzfläche frei von Dispersionströpfchen zu erkennen ist (vgl.: "Dimensionierung liegender Flüssig-Flüssig-Abscheider anhand diskontinuierlicher Absetzversuche", Dipl. Ing. Martin Henschke, VDI-Verlag, Düsseldorf, 1995).

Unter dem Begriff "korrespondierendes Alkan" wird hier und im folgenden die Verbindung verstanden, die identisch mit der Verbindung ist, die durch vollständige Hydrierung des als Ausgangsprodukt verwendeten Alkens hergestellt würde.
Auch wenn die Obergrenze des korrespondierenden Alkans an sich nicht für die Verringerung der Absetzgeschwindigkeit entscheidend ist, wird bevorzugterweise für die Lösung der Aufgabe nur eine geringe Menge des korrespondierenden Alkans verwendet, im Allgemeinen nicht mehr als 10 Gew.-%, insbesondere um eine akzeptable Raum-Zeit-Aausbeute zu gewährleisten. Das korrespondierende Alkan fungiert erfindungsgemäß nicht als Lösemittel.
Gemäß einer besonders bevorzugten Ausführungsform der vorliegenden Erfindung sind die Mengen jedoch so klein, dass die Raum-Zeit-Ausbeute der Epoxidierung nur unwesentlich verringert wird.

Eine weitere, ebenfalls bevorzugte Form der erfindungsgemäßen Verwendung besteht darin, dass die Expodierung kontinuierlich betrieben wird.

Sofern zur verbesserten Reaktionsführung ein Phasentransferkatalysator benötigt wird, ist der Einsatz oder die Gegenwart eines Katalysatorsystems, das mindestens ein Metall der Gruppen IVb, Vb oder VIb des Periodensystems der Elemente darstellt oder enthält, erfindungsgemäß bevorzugt.-Weitere Bestandteile des Katalysatorsystems können Phosphorsäure und mindestens ein quartäres Ammoniumsalz oder ein tertiäres Amin sein. Es können aber auch je nach gewünschter Reaktionsführung Mischungen dieser Katalysatorsysteme verwendet werden. Bei Verwendung eines solchen Katalysatorsystems wird der Reaktionsmischung zur optimalen Reaktionsführung vorzugsweise Phosphorsäure und mindestens ein quartäres Ammoniumsalz hinzugegeben.

Der für die Epoxidierung bevorzugt eingesetzte Katalysator ist ein Metall der Gruppen IVb, Vb und VIb des Periodischen Systems, das in metallischer Form oder in Form eines Komplexes entsprechend dem Oxidationsgrad Null eingesetzt werden kann, oder in dem es sich mit einem variablen Oxidationsgrad befindet. Besonders bevorzugt werden Molybdän, Wolfram, Vanadium, Chrom und Titan.

Unter den anorganischen Derivaten dieser Elemente kann man die Oxide, die gemischten Oxide, die Hydroxide, Oxysäuren, Heteropolysäuren, deren Salze und Ester, die Salze, die von Wasserstoffsäuren und von anorganischen Oxysäuren und organischen Carboxyl- oder Sulfonsäuren mit nicht mehr als 20 Kohlenstoffatomen stammen, deren Anionen unter den Reaktionsbedingungen stabil sind, verwenden.
Als Beispiele für entsprechende Katalysatoren sind zu nennen: Molybdän, Wolfram, Chrom, Vanadium, Titan, die carbonylierten Metalle Mo(CO)₆, W(CO)₆, die Oxide MoO₂, Mo₂O₅, Mo₂O₃, MoO₃, WO₂, W₂O₅, WO₆, CrO₂, Cr₂O₃, CrO₃, VO₂, V₂O₅, ZrO₂, TiO, TiO₂, Ti₂O₃, NbO₂, Nb₂O₃, Nb₂O₅, die Sulfide MoS₂, MoS₃, MoS₄, Mo₂S₃, Mo₂S₅, die Oxychloride von Molybdän, Wolfram, Chrom, Vanadium, Zirkonium, Titan, die Fluoride, Chloride, Bromide, Iodide, Nitrate, Sulfate, Phosphate, Pyrophosphate, Polyphosphate, Borate, Carbonate, Formiate, Octanoate, Dodecanoate, Naphthenate, Stearate, Oxalate, Succinate, Glutarate, Adipate, Benzoate, Phthalate, Benzolsulfonate von Molybdän, Wolfram, Titan, Chrom, Zirkonium, Vanadium, Komplexe wie die Acetylacetonate und Phthalocyanine; die Molybdän-, Wolfram-, Vanadin-, Chromsäuren, die entsprechenden Heteropolysäuren, wie die Phosphormolybdän-, -wolfram-, Arsenmolybdän-, -wolframsäuren ebenso wie alle Alkali- oder Erdalkalisalze dieser Säuren.

In einer besonders bevorzugten Ausführungsform der vorliegenden Erfindung wird ein Wolframkatalysator unter Zusatz von Phosphorsäure verwendet. Die Wolframverbindung wird bevorzugt in einer Konzentrationen von 0,01 bis 0,5 Mol-%, bezogen auf das Alken, eingesetzt. Der Anteil an Phosphorsäure beträgt üblicherweise 0,1 bis 5 Mol-%, bezogen auf die molare Menge an eingesetztem Wolfram.

Die verwendeten quartären Ammoniumsalze entsprechen der allgemeinen Formel

[NR₁R₂R₃R₄]⁺X⁻

wobei R₁, R₂, R₃ und R₄, unabhängig voneinander, lineare oder verzweigte Alkylketten mit 1 bis 20 Kohlenstoffatomen oder Arylgruppen mit 6 bis 10 Atomen darstellen. Die Alkyl- oder Arylgruppen können optional mit weiteren organischen Gruppen oder Atomen, wie zum Beispiel Halogenen, substituiert sein. X- ist ein Gegenion für das Ammoniumion, wie zum Beispiel Chlorid, Bromid, Fluorid, Iodid, Hydrogensulfat, Acetat, Propionat oder Formiat.

Die tertiären Amine haben 3 Alkylreste, die - gleich oder verschieden - in Summe mindestens 18 Kohlenstoffatome aufweisen.

Im Allgemeinen wird als Katalysator bevorzugt ein homogener Katalysator verwendet. Es ist aber auch möglich, einen heterogenen Katalysator zu verwenden, bei dem die katalytisch wirksamen Bestandteile auf einem Trägermaterial, wie beispielsweise Aluminiumoxid, Siliziumdioxid, Aluminiumsilikat, Zeolithen oder geeigneten Polymeren in an sich bekannter Weise aufgebracht sind.
Die besten Ergebnisse im Hinblick auf die beschriebene Epoxidierung wurden mit einem Katalysator erhalten, der in der DE 30 27 349 beschrieben ist. Dieser Katalysator besteht aus einer ersten Komponente aus mindestens einem Element oder mindestens einem seiner anorganischen, organischen oder metallorganischen Derivate aus der Gruppe von W, Mo, V, vorzugsweise W, das in situ und bei Reaktionsbedingungen in eine katalytisch aktive Verbindung umgewandelt werden kann.
Ein besonders geeignetes System besteht aus 0,2 mol % Natriumwolframat bezogen auf Cycloalken, 0,2 mol % Phasentransferkatalysator Trioctylmethylammoniumchlorid bezogen auf Cycloalken, 0,1 mol % Phosphorsäure bezogen auf Cycloalken, und einer Menge Schwefelsäure, die den pH-Wert auf 3 einstellt.

Das Reaktionsgemisch enthält mindestens zwei flüssige Phasen. Davon ist die eine Phase eine wässrige Phase, in der das Wasserstoffperoxid gelöst ist. Dadurch entfällt der Einsatz polarer Lösemittel und der damit einhergehende Verlust an Raum-Zeit-Aausbeute sowie der erhöhte Trennungsaufwand.

Der pH-Wert sollte gemäß einer weiteren, bevorzugten Ausführungsform während der Reaktion konstant gehalten werden. Dies kann durch eine automatische pH-Wert Regelung erreicht werden. Als günstiger pH-Bereich wird erfindungsgemäß ein pH-Wert zwischen 2 und 6 gesehen. Besonders bevorzugt wird die Reaktion bei einem pH-Wert zwischen 2,5 und 4 durchgeführt.
Als cyclisches, mindestens einfach ungesättigtes Alken können alle geeigneten Verbindungen eingesetzt werden.
Diese Alkene können gegebenenfalls durch funktionelle Gruppen substituiert sein, die in dem Reaktionsmilieu stabil sind, wie zum Beispiel Hydroxy-, Chlor-, Fluor-, Brom-, Iod-, Nitro-, Alkoxy-, Amino-, Carbonyl-, Säure-, Ester-, Amid- oder Nitril-Reste.
Sie können aber auch mehrfach ungesättigt sein, wie zum Beispiel Diene, Triene in konjugierter oder nichtkonjugierter Form.

Besonders bevorzugt eignet sich der erfindungsgemäße Absetzbeschleuniger zur Epoxidierung cyclischer, mindestens einfach ungesättigter Alkene, die 8 bis 20 Kohlenstoffatome im Cyclus aufweisen. In diesem Zusammenhang sind insbesondere Cycloocten, Cyclooctadien, Cyclododecen, Cyclododecadien, Cyclododecatrien, Dicyclopentadien und Cyclododecen zu nennen. Vor allem solche Alkene mit mehr als neun Kohlenstoffatomen lassen sich im Hinblick auf die Raum-Zeit-Ausbeute wesentlich einfacher darstellen, verglichen mit den Verfahren des Standes der Technik.
Ganz besonders bevorzugt ist die Verwendung zur Herstellung cyclischer Epoxyalkane, insbesondere zur Herstellung von 1,2-Epoxycyclododecan.

Unter dem zum eingesetzten cyclischen, mindestens einfach ungesättigten Alken korrespondierenden Alkan versteht man die cyclische, gesättigte organische Verbindung mit der gleichen Anzahl von Kohlenstoffatomen. Die korrespondierenden Verbindungen sind dann beispielsweise ausgewählt aus der Gruppe von Cyclooctan, Cyclododecan und Dicyclopentan.

Das beschriebene, kontinuierliche Epoxidierungsverfahren kann in einem oder- mehreren Reaktoren durchgeführt werden. Ein Beispiel für eine kaskadenartige Reaktionsführung über mehrere Stufen wird in der EP 1 167 334 A2 beschrieben. Dort wird die Reaktionsmischung in die vordere Reaktionszone eingebracht und durchläuft mehrere nachgeschaltete Reaktionszonen, bis die das Produkt enthaltende Mischung schließlich an der letzten Reaktionszone abgeführt wird. Dabei können die einzelnen Reaktionszonen in unterschiedlichen Reaktoren oder in einem Reaktor integriert sein.

Besonders bevorzugt wird der Absetzbeschleuniger bei Epoxidierungsverfahren unter Verwendung eines Phasentransferkatalysators eingesetzt, in denen die Ausgangsmischung aus Alken und korrespondierendem Alkan zusammen mit dem quartären Ammoniumsalz in einen ersten Reaktor dosiert werden. In diesen Reaktor wird eine dem Alken entsprechende molare Menge an Wasserstoffperoxid dosiert, welches bereits Phosphorsäure und den in der DE 30 27 349 beschriebenen Katalysator auf Wolframbasis enthält. Es ist möglich, die Menge von Wasserstoffperoxid um 20 % zu über- oder zu unterschreiten. Ab einem gewissen Füllungsgrad des ersten Reaktors, der einer bestimmten Verweilzeit entspricht, wird die Lösung entweder über Überlauf oder über eine Pumpe kontinuierlich in einen weiteren Reaktor gefördert. In diesen zweiten Reaktor wird gegebenenfalls eine kleine Menge Wasserstoffperoxid ohne Katalysator und Phosphorsäure dosiert. Ab einem gewissen Füllungsgrad des zweiten Reaktors, der einer bestimmten Verweilzeit entspricht, wird die Lösung entweder über Überlauf oder über eine Pumpe kontinuierlich in einen weiteren Reaktor gefördert. In diesen dritten Reaktor wird gegebenenfalls eine kleine Menge Wasserstoffperoxid ohne Katalysator und Phosphorsäure dosiert. Diese Form der Reaktorkaskade kann über 2 bis 8 Stufen geführt werden. Diese Stufen können auch in einem Reaktor integriert werden, in dem die Mehrstufigkeit des Reaktors über entsprechende Einbauten realisiert wird.
Das Wasserstoffperoxid kann je nach angestrebtem Umsatz auf die einzelnen Reaktoren aufgeteilt werden. Das verwendete Wasserstoffperoxid ist handelsübliches Wasserstoffperoxid mit Konzentrationen von 10 bis 70 % Wasserstoffperoxid in Wasser.
Nach beendeter Reaktion und vollständiger Phasentrennung kann das Wolfram aus der organischen durch, optional mehrfache, Extraktion mit Wasser, welches gegebenenfalls basische Zusätze wie zum Beispiel Natriumcarbonat, Natriumhydrogencarbonat oder Natriumhydroxid enthält, extrahiert werden. Danach kann das Alkan, zusammen mit Resten von nicht umgesetztem Alken abdestilliert werden. Aus dem Sumpf dieser Destillation wird das reine Epoxid durch Feindestillation gewonnen. Die Vorbehandlung dient einer Reinigung vor der Destillation, um so die Entstehung von Nebenprodukten zu verringern.

Die Temperatur bei der Epoxidierung kann zwischen 50 und 120 °C liegen. Bevorzugt sind Temperaturen zwischen 70 und 100 °C.

Die Epoxidierung wird bevorzugt unter Schutzgas (Stickstoff, Argon, Kohlendioxid) durchgeführt.

Die folgenden Beispiele dienen der Erläuterung der Verwendung des Absetzbeschleunigers, ohne diese darauf zu beschränken.

### Beispiel 1 (Vergleichsbeispiel):

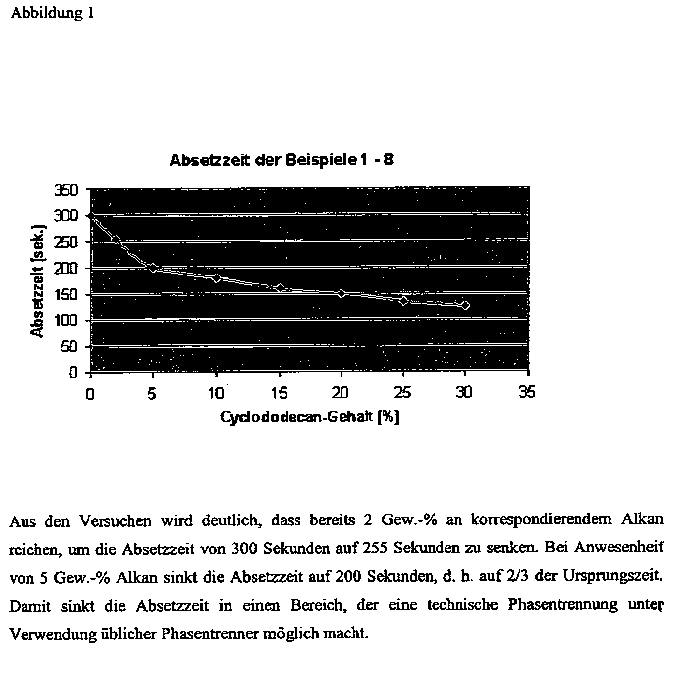

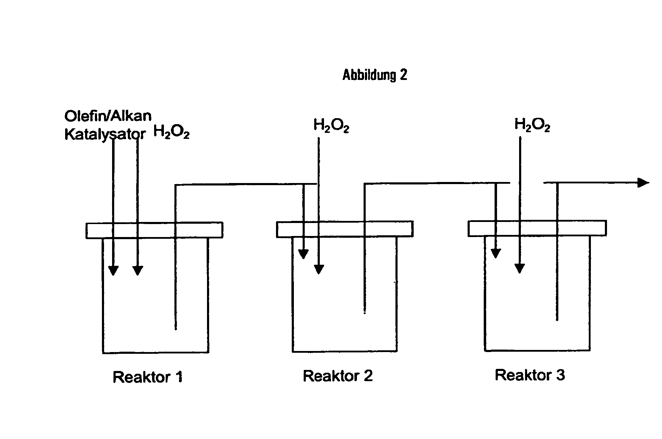

In einer Apparatur analog Abbildung 2 wurden in Reaktor 1 249 g Cyclododecen (1,5 Mol), 0,99 g Natriumwolframat, 0,59 g Phosphorsäure, 14 g Wasser, 1,2 g Aliquat 336 (Cognis) und 10,2 g Wasserstoffperoxid vorgelegt und mit Schwefelsäure auf pH 3 gebracht. Dann wurde auf 90 °C aufgeheizt und über 2 Stunden 102 g 50 %-ige Wasserstoffperoxidlösung zudosiert. In Reaktor 1 wurde ein Umsatz des Cyclododecens von etwa 90 % erreicht. Das Volumen betrug etwa 300 ml. Von nun an wurde kontinuierlich über 4 Stunden 2,7 ml/min einer Mischung aus Cyclododecen mit 0,2 Mol% Aliquat 336 und mit 0,73 ml/min einer Lösung aus 0,94 % Natriumwolframat, 0,56 % Phosphorsäure und 98,5 % Wasserstoffperoxid (50 %-ig) zudosiert. Über einen Überlauf gelangte die Mischung kontinuierlich in Reaktor 2, der ebenfalls ein Volumen von 400 ml hatte und bei 90 °C betrieben wurde. Nach vier Stunden kontinuierlicher Fahrweise erreichte der Umsatz im Reaktor 3 99,6 %.

Nach der Reaktion wurde die Absetzzeit der Mischung in Reaktor 3 bestimmt. Die Absetzzeit ist die Zeit nach der die wässrige Phase so abgesetzt war, das die zweiphasige Schicht am Rand des Reaktors kleiner als 1 mm betrug. Dabei wurde die Absetzzeit alle 15 Sekunden kontrolliert. Das bestimmen der Absetzzeit wurde 2 Mal wiederholt und ein Mittelwert gebildet. Die Ergebnisse sind in Tabelle 1 dargestellt.

### Beispiel 2 (erfindungsgemäßes Beispiel):

Der Versuch wurde analog Beispiel 1 durchgeführt, nur wurde dem Edukt 2 % Cyclododecan bezogen auf Cyclododecen zugegeben. Die Ergebnisse sind in Tabelle 1 dargestellt.

### Beispiel 3 (erfindungsgemäßes Beispiel):

Der Versuch wurde analog Beispiel 1 durchgeführt, nur wurde dem Edukt 5 % Cyclododecan bezogen auf Cyclododecen zugegeben. Die Ergebnisse sind in Tabelle 1 dargestellt.

### Beispiel 4 (erfindungsgemäßes Beispiel):

Der Versuch wurde analog Beispiel 1 durchgeführt, nur wurde dem Edukt 10 % Cyclododecan bezogen af Cyclododecen zugegeben. Die Ergebnisse sind in Tabelle 1 dargestellt.

### Beispiel 5 (erfindungsgemäßes Beispiel):

Der Versuch wurde analog Beispiel 1 durchgeführt, nur wurde dem Edukt 15 % Cyclododecan bezogen auf Cyclododecen zugegeben. Die Ergebnisse sind in Tabelle 1 dargestellt.

### Beispiel 6 (erfindungsgemäßes Beispiel):

Der Versuch wurde analog Beispiel 1 durchgeführt, nur wurde dem Edukt 20 % Cyclododecan bezogen auf Cyclododecen zugegeben. Die Ergebnisse sind in Tabelle 1 dargestellt.

### Beispiel 7 (erfindungsgemäßes Beispiel):

Der Versuch wurde analog Beispiel 1 durchgeführt, nur wurde dem Edukt 25 % Cyclododecan bezogen auf Cyclododecen zugegeben.

### Beispiel 8 (erfmdungsgemäßes Beispiel):

Der Versuch wurde analog Beispiel 1 durchgeführt, nur wurde dem Edukt 30 % Cyclododecan bezogen auf Cyclododecen zugegeben.

**Tabelle 1:**

| Beispiel Nr. | Cyclododecangehalt [in Gew.-%] | Absetzzeit [in Sekunden] |
|---|---|---|
| 1 (Vergleich) | 0 | 300 |
| 2 | 2 | 255 |
| 3 | 5 | 200 |
| 4 | 10 | 180 |
| 5 | 15 | 160 |
| 6 | 20 | 150 |
| 7 | 25 | 135 |
| 8 | 30 | 125 |

## Patentansprüche

1. Verwendung eines cyclischen Alkans mit 8 bis 20 Kohlenstoffatomen im Cyclus als Absetzbeschleuniger bei der Epoxidierung eines cyclischen, mindestens einfach ungesättigten Alkens mit 8 bis 20 Kohlenstoffatomen im Cyclus, wobei als cyclisches Alkan die zu dem eingesetzten Alken korrespondierende, gesättigte Verbindung verwendet wird, **dadurch gekennzeichnet, dass** die Epoxidierung in Gegenwart von Wasserstoffperoxid als Oxidationsmittel und eines Katalysatorsystems durchgeführt wird, und wobei das Katalysatorsystem mindestens ein Metall der Gruppen IVb, Vb oder VIb des Periodensystems der Elemente, Phosphorsäure sowie einen Phasentransferkatalysator enthält, dass die Menge an cyclischem Alkan in der Reaktionsmischung mindestens 1 Gew.-% beträgt und dass das Reaktionsgemisch mindestens zwei flüssige Phasen enthält.

2. Verwendung nach Anspruch 1,
**dadurch gekennzeichnet,**
**dass** die Menge an cyclischem Alkan in der Reaktionsmischung mindestens 2,5 Gew.-% beträgt.

3. Verwendung nach einem der vorhergehenden Ansprüche,
**dadurch gekennzeichnet,**
**dass** die Epoxidierung kontinuierlich durchgeführt wird

4. Verwendung nach einem der vorhergehenden Ansprüche,
**dadurch gekennzeichnet,**
**dass** der pH-Wert des Reaktionsgemisches zwischen 2 und 6, bevorzugt zwischen 2,5 und 4 gehalten wird.

5. Verwendung nach einem der vorhergehenden Ansprüche,
**dadurch gekennzeichnet,**
**dass** das cyclische Alkan Cyclododecan und das cyclische Alken Cyclododecen sind.

## Claims

1. The use of a cyclic alkane having from 8 to 20 carbon atoms in the ring as settling accelerator in the epoxidation of a cyclic, at least monounsaturated alkene having from 8 to 20 carbon atoms in the ring, the saturated compound corresponding to the alkene used being used as the cyclic alkane, **characterized in that** the epoxidation is carried out in the presence of hydrogen peroxide as oxidant and a catalyst system, and the catalyst system containing at least one metal of groups IVb, Vb or VIb of the Periodic Table of the Elements, phosphoric acid and a phase transfer catalyst, **in that** the amount of cyclic alkane in the reaction mixture is at least 1% by weight and **in that** the reaction mixture contains at least two liquid phases.

2. The use according to claim 1, **characterized in that** the amount of cyclic alkane in the reaction mixture is at least 2.5% by weight.

3. The use according to either one of the preceding claims, **characterized in that** the epoxidation is carried out continuously.

4. The use according to any one of the preceding claims, **characterized in that** the pH of the reaction mixture is maintained at a value in the range from 2 to 6, preferably from 2.5 to 4.

5. The use according to any one of the preceding claims, **characterized in that** the cyclic alkane is cyclododecane and the cyclic alkene is cyclododecene.

## Revendications

1. Utilisation d'un alcane cyclique comportant 8 à 20 atomes de carbone dans le cycle en tant qu'accélérateur de la décantation lors de l'époxydation d'un alcène cyclique au moins mono-insaturé comportant 8 à 20 atomes de carbone dans le cycle, en utilisant, comme alcane cyclique le composé saturé correspondant à l'alcène mis en oeuvre,
**caractérisé en ce que**
l'époxydation est réalisée en présence de peroxyde d'hydrogène en tant qu'agent d'oxydation et d'un système catalyseur contenant au moins un métal des groupes IVb, Vb ou VIb de la classification périodique des éléments, de l'acide phosphorique ainsi qu'un catalyseur de transfert de phase, et la quantité d'alcane cyclique dans le mélange réactionnel est au moins égale à 1 % en poids, et le mélange réactionnel contient au moins deux phases liquides.

2. Utilisation selon la revendication 1,
**caractérisée en ce que**
la quantité d'alcane cyclique, dans le mélange réactionnel, est au moins égale à 2,5 % en poids.

3. Utilisation selon au moins l'une quelconque des revendications précédentes,
**caractérisée en ce que**
l'ëpoxydation est réalisée en continu.

4. Utilisation selon au moins l'une quelconque des revendications précédentes,
**caractérisée en ce que**
le pH du mélange réactionnel est maintenu entre 2 et 6, de préférence entre 2,5 et 4.

5. Utilisation selon au moins l'une quelconque des revendications précédentes,
**caractérisée en ce que**
l'alcane cyclique est du cyclododécane et l'alcène cyclique du cyclododécène.
